# EUROPÄISCHE PATENTSCHRIFT

(11) **EP 1 061 968 B1**
(45) Veröffentlichungstag und Bekanntmachung des Hinweises auf die Patenterteilung: **10.08.2005**
(21) Anmeldenummer: 99903662.7
(22) Anmeldetag: 20.01.1999
(51) Int. Cl.: A61M 1/10, F04D 29/18

(54) **INTRAVASAL EINFÜHRBARE SELBSTENTFALTBARE AXIALPUMPE ZUR TEMPORÄREN HERZUNTERSTÜTZUNG**
SELF-DEPLOYING AXIAL-FLOW PUMP INTRODUCED INTRAVASCULARLY FOR TEMPORARY CARDIAC SUPPORT
POMPE AXIALE AUTODEPLOYABLE INSERABLE PAR VOIE INTRAVASCULAIRE POUR ASSISTANCE CARDIAQUE TEMPORAIRE

(30) Priorität: 07.03.1998 DE 29804046 U
(43) Veröffentlichungstag der Anmeldung: 27.12.2000
(73) Patentinhaber: Schmitz-Rode, Thomas, 52070 Aachen (DE); Günther, Rolf W., Prof. Dr., 52074 Aachen (DE)
(72) Erfinder: Schmitz-Rode, Thomas, 52070 Aachen (DE); Günther, Rolf W., Prof. Dr., 52074 Aachen (DE)
(74) Vertreter: Selting, Günther, Dipl.-Ing.
(86) Internationale Anmeldenummer: PCT/EP1999/000368
(87) Internationale Veröffentlichungsnummer: WO 1999/044651

(56) Entgegenhaltungen:
- EP-A- 0 364 293
- WO-A-94/05347
- US-A- 4 753 221

## Beschreibung

Die Erfindung betrifft eine intravasal einführbare selbstentfaltbare Axialpumpe zur temporären Herzunterstützung.

Im kardiogenen Schock ist die Auswurfleistung des linken Ventrikels erheblich reduziert. Die verminderte Koronarversorgung kann zum irreversiblen Herzyersagen führen. Durch den Einsatz eines temporären linksventrikulären Unterstützungssystems soll die Pumpfunktion des linken Ventrikels teilweise bzw. weitgehend übernommen und die Koronarperfusion verbessert werden. Bei Herzoperationen kann ein solches System links- und rechtsventrikulär eingesetzt werden und eine Herz-Lungenmaschine ersetzen.

Ein perkutan implantierbares System, das bisher klinische Bedeutung erlangt hat, ist die intraaortale Ballongegenpulsation (IABP). Die erzielbare hämodynamische Verbesserung ist jedoch nur sehr begrenzt.

Eine intravasal einführbare Axialpumpe, von der der Oberbegriff des Patentanspruchs 1 ausgeht, ist in US 4,753,221 beschrieben. Diese Vorrichtung weist ein Rohr mit einem flexiblen trichterförmigen Teil auf, der radial zusammengedrückt und in einen Katheter eingebracht werden kann, welcher den trichterförmigen Teil in eingeschnürtem Zustand hält. Der trichterförmige Teil des Rohres enthält einen Rotor, der axial komprimiert werden kann und mit einer Antriebswelle verbunden ist.

In EP 0 364 293 A2 ist ein Blutpumpenkatheter beschrieben, der ebenfalls ein Rohr mit einem elastisch radial aufweitbaren Teil aufweist. In dem radial aufweitbaren Teil, das stromab von der Aortenklappe in der Aorta platziert wird, befindet sich ein deformierbarer Rotor, der sich nach dem Zurückziehen eines Führungsrohres oder eines Katheters entfaltet.

Eine bekannte transfemoral implantierbare Mikro-Axialpumpe "Hemopump™" stellt sich nach experimenteller und vorläufiger klinischer Prüfung als erfolgversprechendes Konzept dar, welches eine ausreichende Linksherzentlastung bewirken kann. Der Ansaugstutzen der Pumpe wird retrograd über die Aortenklappe im linken Ventrikel plaziert. Der Pumpenrotor befindet sich am Ende einer Kanüle in der oberen Aorta descendens und wird durch einen externen Motor angetrieben. Nachteil des Systems ist, daß die transfemorale Implantation aufgrund des großen Durchmessers des Motors nur operativ über eine femorale Arteriotomie und gegebenenfalls eine Graftankopplung möglich ist.

Der vorliegenden Erfindung liegt die Aufgabe zugrunde, eine alternative Konstruktion für den Rotor einer intravasal einführbaren Axialpumpe zu schaffen.

Zur Lösung dieser Aufgabe wird ein aus radial komprimierbaren bzw. entfaltbaren Komponenten aufgebautes System mit den im Anspruch 1 angegebenen Merkmalen vorgeschlagen.

Die radiale Komprimierbarkeit der Komponenten erlaubt die Realisierung eines für eine perkutane Implantation in Seldinger-Technik vertretbar kleinen Punktionsdurchmessers. Durch die Entfaltung im Herz-Gefäßsystem kann ein relativ großer Pumpendurchmesser von 10 bis 14 mm vorgesehen werden. Hierdurch sinkt die Rotordrehzahl und damit die mechanische Beanspruchung der Komponenten.

Im folgenden wird unter Bezugnahme auf die Zeichnungen ein Ausführungsbeispiel der Erfindung näher erläutert.

Es zeigen :
- Fig. 1: eine parasagittale Ansicht der Axialpumpe nach ihrer Verlegung über die Aortenklappe mit der Spitze in den linken Ventrikel eines Herzens,
- Fig. 2a: eine vergrößerte Darstellung des distalen Endbereichs des Rohres,
- Fig. 2b: eine vergrößerte Darstellung des proximalen Endbereichs des Rohres, und
- Fig. 3: eine Darstellung des entfalteten Rotors.

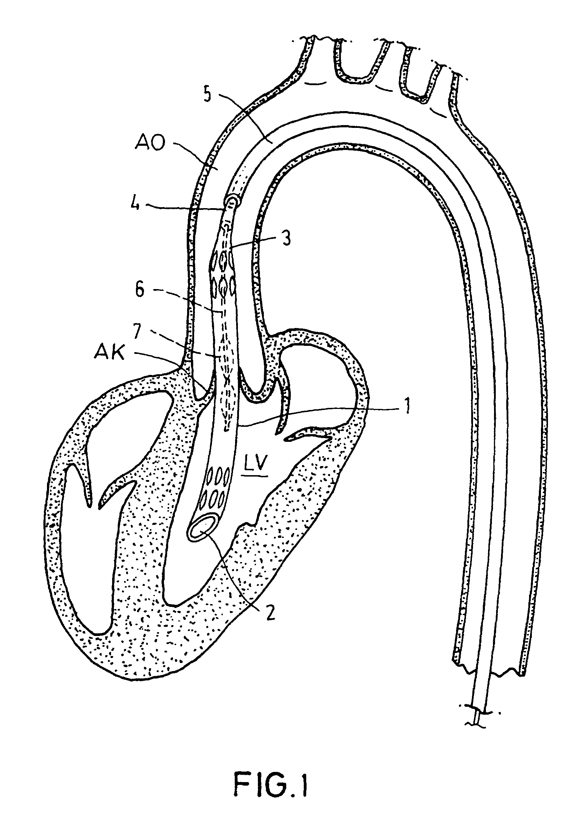

Fig. 1 zeigt das positionierte Pumpensystem zur Linksherz-Unterstützung. Das Ansaugrohr 1 befindet sich mit der Spitze 2 in der linken Herzkammer LV. Das distale Ende 3 liegt in der Aorta ascendens AO und geht in einen Katheter 4 über, der von einem Deckschlauch 5 umgeben ist. Katheter und Deckschlauch führen über die Punktionsstelle in der Leistenarterie nach außen. Der Katheter 4 enthält eine von außen mit einem Motor angetriebene flexible rotierbare Welle 6, an deren Spitze sich ein selbstentfaltbarer Rotor 7 befindet. Der Rotor 7 liegt innerhalb des Rohres 1. Der Außendurchmesser des entfalteten Rotors 7 ist minimal geringer als der Innendurchmesser des entfalteten Rohres 1, so daß der Rotor 7 nahezu den gesamten Rohrquerschnitt ausfüllt und unter Rotation in dem Rohr 1 geführt wird. Zur Positionierung des Systems sind Rohr 1 und Rotor 7 radial komprimiert und durch den bis zur Rohrspitze 2 vorgeschobenen rohrförmigen Deckschlauch 5 überdeckt. In dieser Konfiguration wird das System perkutan in Seldinger-Technik über einen Führungsdraht mit der Spitze durch die Aortenklappe AK hindurch bis in den linken Ventrikel LV vorgeschoben. Die Entfaltung erfolgt, indem der Deckschlauch 5 auf dem fixierten Katheter 4 zurückgezogen wird, bis die Spitze des Deckschlauchs 5 das Rohrende 3 freigegeben hat. Zur Entfernung des Systems wird der Deckschlauch 5 bis zur Rohrspitze 2 vorgeschoben, wodurch Rotor 7 und Rohr 1 in komprimiertem Zustand in den Deckschlauch 5 eingezogen werden, wonach dieser durch die Punktionsstelle hindurch extrahiert wird.

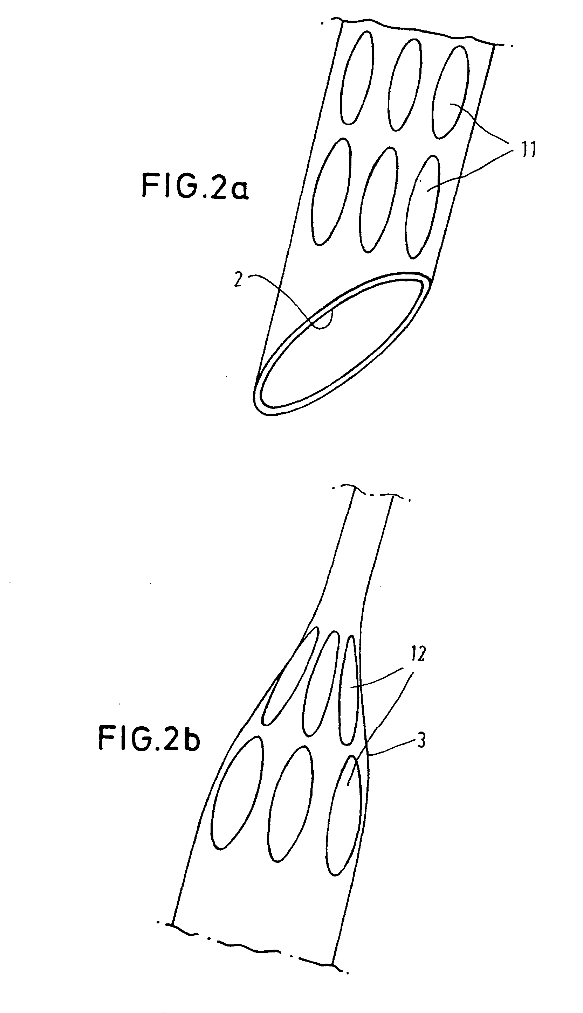

Fig. 2 zeigt Gestaltungsdetails des Rohres 1. Die Spitze 2 kann schräg angeschnitten sein, um den Ansaugquerschnitt zu vergrößern (Fig. 2a). Zusätzlich können Seitenlöcher 11 vorliegen. Das sich verjüngende Ende 3 des Rohres weist mehrere Austrittslöcher 12 auf, die rund oder schlitzförmig gestaltet sein können (Fig. 2b). Das Rohr 1 besteht vorzugsweise aus kunststoffbeschichtetem selbstexpandierbarem metallischem Endoprothesenmaterial mit einem Durchmesser von 10 bis 14 mm und einer Länge von 7 bis 12 cm nach Entfaltung.

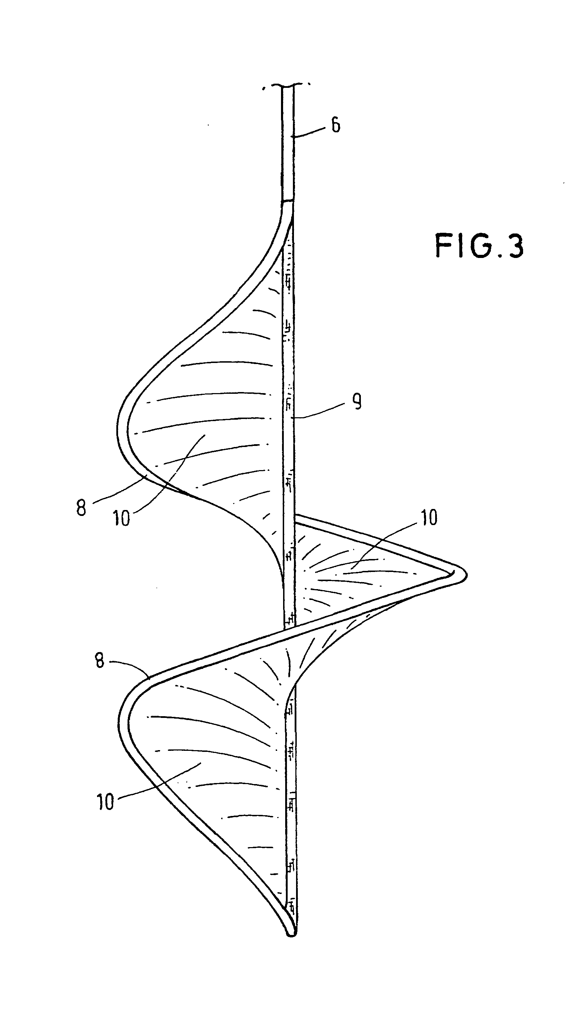

Fig. 3 zeigt ein Gestaltungsbeispiel des entfaltbaren Rotors 7 in Form einer Archimedes-Spirale. Diese besteht aus einer Wendel 8 aus Memory-Metall (Nitinol), deren beide spitzwinklig in die Mittelachse auslaufenden Enden durch ein elastisches Band 9 verbunden sind. Das Band 9 verläuft in der Mittelachse (Rotationsachse) der Wendel 8. Die helixförmige Rotorschaufel wird durch eine elastische Bespannung 10 aus einem spongiösen Kreuzgewebe gebildet, die sich zwischen Wendel 8 und Band 9 erstreckt. Als Material für die Bespannung eignet sich vorzugsweise eine netzförmige hochelastische Kunststoffmatrix, die mit einer dünnen Silikon- oder Polyurethanhaut überzogen ist. Die Orientierung der Bespannungsfläche ist dadurch definiert, daß ein bestimmter Punkt auf der Wendel 8 mit dem jeweils nächstliegenden Punkt auf dem Band 9 (Rotationsachse) geradlinig in Verbindung steht.

Minimal besteht der Rotor aus einer Vollwindung (360°) der Wendel, zuzüglich des spitzwinkligen Wendelauslaufs zu beiden Enden (Fig. 3). Sie kann aber auch aus 1_{1/2}, 2 oder mehreren Vollwindungen der Wendel bestehen.

Im komprimierten Zustand verläuft der Wendel-Draht 8 langgestreckt in der Mittelachse, umgeben von dem komprimierten Rohr 1 innerhalb des Deckschlauchs 5. Das elastische Band 9 ist maximal gespannt, das elastische Bespannungsgewebe 10 komprimiert. Bei Entfaltung des Rotors 7 (nach Rückzug des Deckschlauchs 5 und Expansion des Rohres 1) verkürzt sich der Wendel-Draht 8 axial und nimmt die helixförmige Konfiguration ein. Dabei verkürzt sich auch das Band 9 und die Bespannung 10 bildet eine glatte Fläche aus.

## Patentansprüche

1. Intravasal einführbare Axialpumpe zur Herzunterstützung, mit einem selbstentfaltbaren und radial komprimierbaren flexiblen Rohr (1) und mit einem im Inneren des Rohres befindlichen selbstentfaltbaren und radial komprimierbaren Rotor (7), wobei das Rohr (1) mit einem Katheter (4) verbunden und der Rotor mit einer in dem Katheter koaxial verlaufenden Antriebswelle (6) verbunden ist,
**dadurch gekennzeichnet,**
**dass** der selbstentfaltbare Rotor (7) aus einer im komprimierten Zustand gestreckten und im entfalteten Zustand wendelförmigen Drahtwendel (8) besteht, deren beide spitzwinklig in die Mittelachse auslaufenden Enden durch ein in der Mittelachse verlaufendes elastisches Band (9) verbunden sind, und sich eine elastische Bespannung (10) zwischen Drahtwendel (8) und Band (9) erstreckt.

2. Axialpumpe nach Anspruch 1, **dadurch gekennzeichnet, dass** das selbstentfaltbare Rohr (1) aus kunststoffbeschichtetem Metallendoprothesenmaterial besteht.

3. Axialpumpe nach Anspruch 1 und 2, **dadurch gekennzeichnet, dass** das Rohr (1) sich an einem Ende trichterförmig verjüngt und mit einem Katheter (4) verbunden ist.

4. Axialpumpe nach einem der Ansprüche 1 bis 3, **dadurch gekennzeichnet, dass** das Rohr (1) an beiden Endbereichen schlitzförmige oder runde Durchbrüche aufweist.

5. Axialpumpe nach Anspruch 1, **dadurch gekennzeichnet, dass** die Wendel (8) des selbstentfaltbaren Rotors (7) mindestens eine 360°-Windung bechreibt.

6. Axialpumpe nach Anspruch 1 oder 2, **dadurch gekennzeichnet, dass** die Wendel (8) des selbstentfaltbaren Rotors (7) aus Memory-Metall besteht.

7. Axialpumpe nach einem der Ansprüche 1 bis 6, **dadurch gekennzeichnet, dass** der Außendurchmesser des selbstentfaltbaren Rotors (7) kleiner als der Innendurchmesser des entfalteten Rohres (1) ist, so dass der Rotor (7) den gesamten Rohrquerschnitt ausfüllt und unter Rotation in dem Rohr (1) geführt wird.

8. Axialpumpe nach einem der Ansprüche 1 bis 8, **dadurch gekennzeichnet, dass** der Rotor (7) an einem oder beiden Enden innerhalb des Rohres (1) zentrisch gelagert ist.

## Claims

1. An axial-flow pump to be introduced intravascularly for cardiac support, comprising a self-deploying and radially compressible flexible tube (1) and with a self-deploying and radially compressible rotor (7) within the tube, the tube (1) being connected with a catheter (4) and the rotor being connected with a drive shaft (6), extending coaxially in the catheter,
**characterized in**
**that** the self-deploying rotor (7) is a wire helix (8) elongated when compressed and helical when deployed, both ends of which terminate in the central axis under an acute angle and are connected by an elastic band (9) extending along the central axis, and an elastic cover (10) extends between the wire helix (8) and the band (9).

2. The axial-flow pump of claim 1, wherein the self-deploying tube (1) consists of metal endoprosthetic material coated with plastic material.

3. The axial-flow pump of claims 1 and 2, wherein one end of the tube (1) tapers in the manner of a funnel and is connected with a catheter (4).

4. The axial-flow pump of one of claims 1 to 3, wherein the tube (1) has slit-shaped or circular openings in both end portions.

5. The axial-flow pump of claim 1, wherein the helix (8) of the self-deploying rotor (7) has at least one winding extending over 360°.

6. The axial-flow pump of claim 1 or 2, wherein the helix (8) of the self-deploying rotor (7) is made of memory metal.

7. The axial-flow pump of one of claims 1 to 6, wherein the outer diameter of the self-deploying rotor (7) is smaller than the inner diameter of the deployed tube (1) so that the rotor (7) fills almost the entire cross section of the tube and is guided rotating in the tube (1).

8. The axial-flow pump of one of claims 1 to 7, wherein the rotor (7) is centrally supported at one or both ends within the tube (1).

## Revendications

1. Pompe axiale insérable par voie intravasculaire pour assistance cardiaque, comprenant un tuyau (1) flexible autodéployable et pouvant être comprimé radialement et un rotor (7) se trouvant à l'intérieur du tuyau, autodéployable et pouvant être comprimé radialement, le tuyau (1) étant relié à un cathéter (4) et le rotor à un arbre d'entraînement (6) s'étendant coaxialement dans le cathéter,
**caractérisée en ce que**
le rotor (7) autodéployable est constitué d'un boudin (8) allongé à l'état comprimé et hélicoïdal à l'état déployé, dont les deux extrémités se terminant en angle aigu dans l'axe médian sont reliées par un ruban (9) élastique s'étendant dans l'axe médian, et **en ce qu'**un voile (10) élastique s'étend entre le boudin (8) et le ruban (9).

2. Pompe axiale selon la revendication 1, **caractérisée en ce que** le tuyau (1) autodéployable est constitué de matériau pour endoprothèse métallique recouvert de matière plastique.

3. Pompe axiale selon la revendication 1 et 2, **caractérisée en ce que** le tuyau (1) se rétrécit en entonnoir à une extrémité et est relié à un cathéter (4).

4. Pompe axiale selon l'une quelconque des revendications 1 à 3, **caractérisée en ce que** le tuyau (1) comporte aux deux zones d'extrémité des ouvertures en forme de fente ou rondes.

5. Pompe axiale selon la revendication 1, **caractérisée en ce que** l'hélice (8) du rotor (7) autodéployable décrit au moins un tour de 360°.

6. Pompe axiale selon la revendication 1 ou 2, **caractérisée en ce que** l'hélice (8) du rotor (7) autodéployable est constituée d'un métal à mémoire.

7. Pompe axiale selon l'une quelconque des revendications 1 à 6, **caractérisée en ce que** le diamètre extérieur du rotor (7) autodéployable est plus petit que le diamètre intérieur du tuyau (1) déployé, de sorte que le rotor (7) occupe la totalité de la section transversale du tuyau et est guidé par rotation dans le tuyau (1).

8. Pompe axiale selon l'une quelconque des revendications 1 à 8, **caractérisée en ce que** le rotor (7) est logé centralement à l'une ou les deux extrémités à l'intérieur du tuyau (1).
